# EUROPEAN PATENT APPLICATION

(11) **EP 1 439 227 A1**
(43) Date of publication of application: **21.07.2004**
(21) Application number: 03000634.0
(22) Date of filing: 15.01.2003
(51) Int. Cl.: C12N 15/11, A61K 31/713, A61P 31/20

(54) **HPV-E6-specific siRNAs for the treatment of HPV-related tumors**

(71) Applicant: Deutsches Krebsforschungszentrum Stiftung des öffentlichen Rechts, 69120 Heidelberg (DE); Cell Center Cologne GmbH, 50931 Köln (DE)
(72) Inventor: Butz, Karin, Dr., 69493 Hirschberg (DE); Hoppe-Seyler, Felix, Prof. Dr., 69118 Heidelberg (DE); Hengstermann, Arnd, 50939 Köln (DE); Scheffner, Martin, 50674 Köln (DE)
(74) Representative: Isenbruck, Günter, Dr.

(57) **Abstract**

Provided is the use of a nucleic acid containing a sequence specific to the HPV18- or HPV16 E7 gene to prepare siRNA which induces apoptosis in HPV-infected cells. The prepared siRNA can be used as a medicament for the treatment of HPV-related tumors.

## Description

The present invention relates to the use of siRNAs which are specific for the HPV E6 oncogene to induce apoptosis in HPV-infected cells by down-regulating E6 expression. Thus, a novel tool for the treatment of HPV-related tumors is provided.

Tumor cells are typically characterized by their failure to undergo so-called programmed cell death or apoptosis, which allows their survival and continuous proliferation under the influence of abnormal growth stimuli. In many virus-associated tumors, anti-apoptotic activities are provided by the functions of viral proteins (Hoppe-Seyler and Butz, 1999, Anticancer Research, Vol. 19, pages 4747-4758). The targeted inhibition of such virus-derived anti-apoptotic factors in tumor cells should, therefore, provide a rational approach towards novel anticancer therapies.

Certain types of human papillomaviruses (HPVs) are closely associated with the development of a variety of different tumors in humans (zur Hausen, 2002, Nature Rev. Cancer, Vol. 2, pages 342-350). Examples include cervical carcinoma, which represents the second most common cancer in females worldwide. The transforming activity of tumor-associated so-called high-risk HPV types, such as HPV16 and HPV18, is dependent on the functions of the viral E6 and E7 oncogenes, the expression of which results in the maintenance of the transformed phenotype of HPV-positive cervical cancer cells. According to one model, E7 acts as a mitotic mutator and growth stimulatory factor that interferes with cellular senescence and proper differentiation. The abnormal growth stimulus exerted by E7 provokes an apoptotic response in HPV-positive cells, which is subsequently counteracted by E6, ultimately resulting in deregulated growth of genetically unstable cells. The functional inhibition of anti-apoptotic viral proteins like the above described E6 protein should enable the specific targeting of virus-associated tumor cells, since these factors are not expressed in uninfected cells.

According to the known state in the art, several strategies have been established to develop effective vaccines based on the inhibition of the expression and/or the function of E6 or E7 which may be useful in treatment and prevention of HPV-associated tumors. One approach exploits synthetic antigenic peptides, which exhibit the disadvantages that it requires large quantities of peptides for vaccines to be effective at a broad spectrum. In a different approach, recombinant microorganisms, e.g. an attenuated strain of *Mycobacterium bovis* (Bacille Calmette-Guerin) and distinct proteins thereof (Heat Shock Protein Hsp65) fused to HPV E7, are used for immune system stimulation of HPV-infected subjects (Chu et al., 2000, Clin. Exp. Immunol., Vol. 121, pages 216-225). However, these vaccine delivery systems have been shown to evoke only weak immune responses. A widespread strategy to immunize for HPV E6- or E7 and, thus, eliminate E6-/E7-exerted propagation of HPV infections is the delivery of so-called virus-like particles (VLP's) bearing usually a viral so-called L1 protein fused to E6 or E7. In general, one problem associated with immunizing subjects with preparations of individual proteins is that most of these proteins are comparatively small and might therefore not comprise many reactive epitopes.

Other approaches focus on interfering with the E6 or E7 expression by the use of specific nucleic acid molecules. Several antisense-based strategies have previously been used to target HPV transcription. However, the efficacy of such approaches was limited in most studies. Usually some degree of growth inhibition rather than induction of apoptosis was reported (Venturini et al., 1999, Nucleic Acids Res., Vol. 27, pages 1585-1592). These differences may be related to the fact that HPV E6/E7 transcripts exhibit low accessibility for antisense nucleic acids.

A rather promising tool to down-regulate and interfere with the expression of proteins has been developed according to the observation that double-stranded RNA molecules (dsRNA) with homology to a specific gene sequence acts as a "trigger" for post-transcriptional gene silencing (PTGS). The unique aspect of this process named "RNA interference" (RNAi) is its exquisite sequence-specificity, leading to the degradation of the targeted mRNA. RNAi has been originally discovered as a mechanism of PTGS in plants and animals (Fire et al., 1998, Nature, Vol. 391, pages 806-811). RNAi can be achieved in mammalian cells following transfection of synthetic 21- and 22-nucleotide siRNAs with overhanging 3' ends (Elbashir et al., 2001, Nature, Vol. 411, pages 494-498), indicating that RNAi may serve as a powerful technology to specifically block the expression of target genes. As such, the use of siRNAs to inhibit the expression of defined target genes has obvious therapeutic potential.

Most recently, a study was published in which HPV 16 E6 and E7 proteins were targeted by the use of specific siRNAs (Jiang and Milner, 2002, Oncogene, Vol. 21, pages 6041-6048). In this study, the application of E6 siRNA caused only a cell growth suppression but no significant cell death, whereas E7 siRNA seem to cause the cells to round up and to undergo apoptosis. But even in the presence of E7 siRNA only a small portion of the cells (approximately 25%) were apoptotic. It is suggested in this study, that E7 siRNA may have a major therapeutic potential for the treatment and possibly the prevention of human cervical cancer. However, considering the relatively weak apoptosis induction, the therapeutic potential of the described E7 siRNAs seem to be rather overestimated. In addition, analysis of the coding-region of the E6 and E7 proteins, respectively, in oncogenic HPVs reveal that E6 and E7 are represented on the same transcript. E6 is expressed from so-called class 1 transcripts which contain the complete E6 open reading frame (ORF). E7 is only expressed from so-called class 2 and class 3 transcripts which are spliced within the ORF of E6 and thus do not express full-length E6. Based on these transcription patterns is not apparent how a selective inhibition of E7 could be achieved by the presented approach.

The applicant has now unexpectedly found that siRNAs being homologous to a distinct HPV18 E6 intron region, that is only present in class 1 transcripts resulted in a dramatic induction of apoptosis in HPV-transformed cells. It is concluded that (i) siRNAs directed against E6 provide excellent molecular tools to specifically and efficiently inhibit intracellular E6 activities, (ii) E6 function is absolutely required to protect HPV-positive tumor cells from apoptosis, and thus (iii) the E6 gene represents a most promising therapeutic target for the treatment of HPV-positive tumors and dysplasias.

Thus, a first aspect of the present invention refers to the use of a nucleic acid containing the sequence of SEQ ID NO:1, a fragment or derivative thereof, to prepare siRNA which induces apoptosis in HPV18-infected cells.

The sequence of SEQ ID NO:1, which is preferably a RNA, corresponds to nucleotides 385 to 399 of the coding sequence of HPV18 E6 as disclosed by the GeneBank Accession Number X05015 (Cole and Danos, 1987, J. Mol. Biol., Vol. 193, pages 599-608).

In a preferred embodiment of the present invention, the nucleic acid containing the sequence of SEQ ID NO:1 has a length of 15 to 25 nucleotides. More preferably, the nucleic acid containing the sequence of SEQ ID NO: 1 has a length of 19 to 22 nucleotides. Most preferred, the nucleic acid containing the sequence of SEQ ID NO: 1 has a length of 19 nucleotides.

In the context of the present invention, the terms "fragment and derivative" refer to nucleic acids that may differ from the original nucleic acid in that they are extended or shortened on either the 5' or the 3' end, on both ends or internally, or extended on one end, and shortened on the other end, provided that the function of the resulting siRNA, namely the down-regulation of the target protein, is not abolished or inhibited. The terms "fragment and derivative" also refer to nucleic acids that may differ from the original nucleic acid in that one or more nucleotides of the original sequence are substituted by other nucleotides and/or (chemically) modified by methods available to the skilled artisan, provided that the function of the resulting siRNA is not abolished or inhibited.

In order to obtain the appropriate length it might be required that the sequence of SEQ ID NO:1 is extended on either the 5' or the 3' terminal end or on both ends, provided that the function of the resulting siRNA, namely the induction of apoptosis, is not inhibited. The extension can occur by any nucleotide or nucleotide sequence. For the present invention, it is preferred that the extension occurs by nucleotides derived from the coding region of the HPV E6 coding region.

Thus, in another embodiment the nucleic acid of the present invention is extended at the 5' end and comprises the sequence of SEQ ID NO:2, a fragment or derivative thereof, corresponding to nucleotides 381 to 399 of Accession Number X05015. In yet another embodiment of the present invention the nucleic acid invention is extended at the 3' end and comprises the sequence of SEQ ID NO:3, corresponding to nucleotides 385 to 403.

In a further aspect, the present invention refers to the use of a nucleic acid containing the sequence of SEQ ID NO:7, a fragment or derivative thereof, to prepare siRNA which induces apoptosis in HPV16-infected cells. SEQ ID NO:7 represents nucleotides 377 of 395 of the coding sequence of HPV16 E6 as disclosed by the GeneBank Accession Number K02718.

If it is necessary or desired for the present invention, any of the nucleotides of SEQ ID NOs:1-3 and 7 can be altered, chemically modified or exchanged by a different nucleotide, assuming that the function of the resulting siRNA, namely the induction of apoptosis, is not inhibited.

The person of skill in the art knows how to prepare siRNA when the above disclosed nucleic acids, particularly RNAs, are provided. Briefly, the strands complementary to the nucleic acids of the present invention are synthesized by any available method and the complementary strands are annealed to the nucleic acid of the present invention under appropriate conditions. The annealing conditions, e.g. temperatures and incubation periods, may be adjusted according to the respective nucleic acid sequence.

In order to exert the desired function, i.e. the induction of apoptosis in HPV-infected cells, the siRNAs prepared from the above nucleic acids of the present invention, are delivered into target cells.

There are several well-known methods of introducing nucleic acids into animal cells, any of which may be used in the present invention and which depend on the host. Typical hosts include mammalian species, such as humans, non-human primates, dogs, cats, cattle, horses, sheep, and the like. At the simplest, the nucleic acid can be directly injected into the target cell / target tissue. Other methods include fusion of the recipient cell with bacterial protoplasts containing the nucleic acid, the use of compositions like calcium chloride, rubidium chloride, lithium chloride, calcium phosphate, DEAE dextran, cationic lipids or liposomes or methods like receptor-mediated endocytosis, biolistic particle bombardment ("gene gun" method), infection with viral vectors, electroporation, and the like.

In a preferred embodiment of the present invention, the siRNAs are delivered to the cell by using liposomes.

The effect of siRNAs, i.e. the reduction of the expression of a certain gene, is considered to be only transient when they are directly applied to cells as described supra. In order to achieve a stable production of siRNAs in HPV-infected cells it can be advantageous if the nucleic acid, preferably a DNA, encoding the respective target siRNA is integrated in an expression vector. Providing suitable elements, as described hereinafter, the DNA is transcribed into the corresponding RNA which is capable of forming the desired siRNA.

The expression vector is preferably a eukaryotic expression vector, or a retroviral vector, a plasmid, bacteriophage, or any other vector typically used in the biotechnology field. If necessary or desired, the nucleic acid can be operatively linked to regulatory elements which direct the synthesis of a mRNA in pro- or eukaryotic cells. Such regulatory elements are promoters, enhancers or transcription termination signals, but can also comprise introns or similar elements, for example those, which promote or contribute to the stability and the amplification of the vector, the selection for successful delivery and/or the integration into the host's genome, like regions that promote homologous recombination at a desired site in the genome. For therapeutic purposes, the use of retroviral vectors has been proven to be most appropriate to deliver a desired nucleic acid into a target cell.

In a preferred embodiment of the present invention, a nucleic acid, preferably a DNA, which is suitable for the preparation of siRNA that induces apoptosis is introduced into a vector which allows for the production of a double-stranded (ds) RNA molecule. Such vectors are known to the person skilled in the art. To drive the expression of siRNAs these vectors usually contain RNA polymerase III promoters, such as the H1 or U6 promoter, since RNA polymerase III expresses relatively large amounts of small RNAs in mammalian cells and terminates transcription upon incorporating a string of 3-6 uridines. Type III promoters lie completely upstream of the sequence being transcribed which eliminates any need to include promoter sequence in the siRNA. If the DNA encoding the desired siRNA should be transcribed from one promoter, the preferred DNA should thus contain the desired coding region of the E6 gene to be targeted as well as its complementary strand, wherein the coding and its complementary strand are separated by a nucleotide linker, allowing for the resulting transcript to fold back on itself to form a so-called stem-loop structure.

An example for such an expression vector which allows for the production of dsRNA directly in the target cell is the so-called pSUPER (Supression of Endogenous RNA). The vector itself and the mechanism how the dsRNA is produced by using said vector is described in Brummelkamp et al., 2002, Science, Vol. 296, pages 550-553. Another example of such a vector named p*Silencer* (Ambion) was developed by Sui et al., 2002, Proc. Natl. Acad. Sci. Vol. 99, pages 5515-5520.

Therefore, another object of the present invention is the use of a nucleic acid, preferably a DNA, containing the sequence of SEQ ID NO:4, a fragment or derivative thereof, to prepare siRNA which induces apoptosis in HPV18-infected cells. SEQ ID NO:4 comprises the DNA corresponding to the RNA depicted in SEQ ID NO: 3, a linker, and the complementary strand to said DNA.

Furthermore, an object of the present invention is the use of a nucleic acid, preferably a DNA, containing the sequence of SEQ ID NO:5, a fragment or derivative thereof, to prepare siRNA which induces apoptosis in HPV18-infected cells. SEQ ID NO:5 comprises the DNA corresponding to the RNA depicted in SEQ ID NO: 2, a linker, and the complementary strand to said DNA.

Furthermore, an object of the present invention is the use of a nucleic acid, preferably a DNA, containing the sequence of SEQ ID NO:8, a fragment or derivative thereof, to prepare siRNA which induces apoptosis in HPV16-infected cells. SEQ ID NO:8 comprises the DNA corresponding to the RNA depicted in SEQ ID NO: 7, a linker, and the complementary strand to said DNA.

The linker is preferably 5 to 15 nucleotides in length, more preferably the linker is 7 to 12 nucleotides long and most preferably it is 9 nucleotides long. The linker can consist of any suitable nucleotide sequence. For the present invention, a linker comprising the nucleotides 20 to 28 of SEQ ID NOs:4, 5, 6 or 8 represents a suitable linker.

It also contemplated in the present invention that the expression of the two complementary strands giving rise to a dsRNA is driven from two promoters, either the same or different. In this case, the nucleotide linker separating the two complementary strands would be omissible. It is further obvious to the one skilled in the art that in this case the DNAs coding for the two complementary siRNA strands can be present on one vector or on two vectors. An example for a vector by which siRNA is expressed from two promoters is described in Lee et al., 2002, Nature Biotechnology, Vol. 19, pages 500-505. For these purposes, nucleic acids containing the nucleotides 1-19 together with 29-47 of either SEQ ID NO: 4, 5, 6 or 8 are inserted into the allocated sites of the vector(s). In the context of the present invention, the nucleic acids containing the nucleotides 1-19 or 29-47 of either SEQ ID NO: 4, 5, 6 or 8 are regarded as fragments or derivatives, according to the above definition, of either SEQ ID NO: 4, 5, 6 or 8.

The vector containing the DNAs of the present invention can be introduced into the target cell by any of the method described above. However, due to several limitations accompanied with the efficient delivery and/or toxic effects, the delivery of the nucleic acids of the present invention can be facilitated by the use of so-called translocating peptides. The use of such peptides are particularly preferred for therapeutic purposes. The peptides are usually linked to the nucleic acid to be delivered in a non-covalent manner.

In general, peptides are contemplated which mimic and act as efficiently as viruses for gene delivery without their limitations of inducing immune responses or being cytotoxic. Examples of peptides forming peptide-DNA complexes for an efficient delivery into a cell comprise DNA-condensing motifs such as polyamines and modifications thereof, active-targeting motifs such as RGD, endosomolytic peptides such as INF, JTS1 or GALA, and nuclear localization sequences (NLS), e.g. derived from the large tumor antigen of Simian 40 virus. An extensive list of translocating peptides and their proposes delivery mechanisms, all of which are contemplated within the scope of the present invention and incorporated herein by reference are described in Morris et al., 2000, Curr. Opin. Biotech., Vol. 8, pages 21 to 27.

The nucleic acids as disclosed in the present invention can be used as a pharmaceutical, optionally in combination with at least one active compound, for the treatment of HPV18-and/or HPV16-related cancers. This is a further embodiment of the present invention. The term "active compound" refers to a compound other than the nucleic acid which is able to induce apoptosis or which inhibits cell proliferation.

Active compounds which are able to induce apoptosis are known to the person skilled in the art. One class of active compounds are chemical compounds having a cytostatic or antineoplastic effect ("cytostatic compound"). Cytostatic compounds included in the present invention comprise, but are not restricted to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18-OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists.

Another class of active compounds which can be used in the present invention are those which are able to induce apoptosis by binding to so-called death receptors ("death receptor ligands"). Other active compounds include agonistic antibodies to death receptors. Another class of active compounds which can be used in combination with the nucleic acid are peptides or proteins which negatively regulate so-called "Inhibitors of Apoptosis Proteins" (IAPs). IAPs bind to early active caspases, thereby preventing the ongoing of the apoptosis process. They are expressed at high levels in many tumors and, by inhibition of caspases, contribute to the resistance of cancers against apoptosis induction.

The nucleic acid can be administered alone or in combination with one or more active compounds. The latter can be administered before, after or simultaneously with the administration of the nucleic acid. The dose of either the nucleic acid or the active compound as well as the duration and the temperature of incubation can be variable and depends on the target that is to be treated.

A further object of the present invention are pharmaceutical preparations which comprise an effective dose of at least one nucleic acid and/or at least one active compound and a pharmaceutically acceptable carrier, i.e. one or more pharmaceutically acceptable carrier substances and/or additives.

The pharmaceutical according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the pharmaceutical compositions can be carried out in a manner known per se. To this end, the nucleic acid and/or the active compound, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiological sodium chloride solution, alcohols such as ethanol, glycerol, polyols, sucrose, invert sugar, glucose, mannitol, vegetable oils, etc. It is also possible to lyophilize the nucleic acid and/or the active compound and to use the resulting lyophilisates, for example, for preparing preparations for injection or infusion. Suitable carriers for microcapsules, implants or rods are, for example, copolymers of glycolic acid and lactic acid.

The pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the nucleic acid, in combination with one or more active compounds to be administered, depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to the nucleic acid.

The nucleic acids according to the present invention, respectively the medicaments containing the latter, can be used for the prophylaxis and/or treatment of any HPV-related disease. Preferably, the medicament of the present invention can be used for the prophylaxis and/or treatment of HPV-16 and/or HPV-18-related diseases. These include anogenital warts such as Condylomata acuminata, Condylomata plana, Condylomata gigantea (Buschke-Loewenstein tumor), Cervical Intraepithelial Neoplasia, Bowenoid papulosis (Bowenoid dysplasia, viral keratosis), mucosa warts such as larynx papilloma, malignant tumors such as spinalioma (accompanied with Epidermodysplasia verruciformis), Morbus Bowen of the skin, penis carcinoma, vulva carcinoma, cervical carcinoma, larynx carcinoma, oropharynx and tongue carcinoma.

In a particular embodiment of the present invention the tumor to be treated is cervical carcinoma.

The invention is further illustrated in the following examples:

### EXAMPLES

### Example 1: siRNA which is specific to the HPV18 E6 intron strongly reduces the levels of HPV18 E6/E7 transcripts

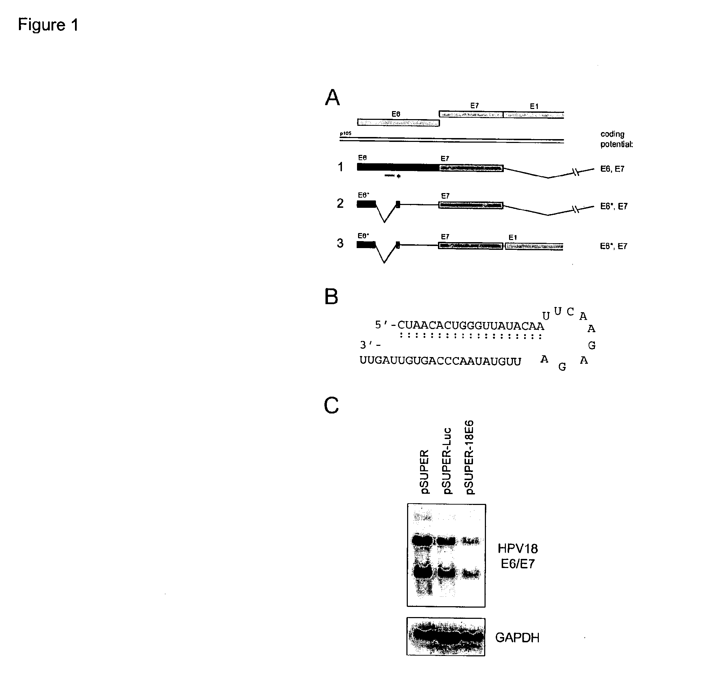

HPV18-positive cancer cell lines typically contain integrated HPV genomes, which exhibit a similar transcription pattern. In general, three classes of transcripts are generated with the potential to encode the viral E6 and E7 proteins (Figure 1A). The E6 protein is generated from class 1 transcripts, which are the only transcripts that contain the complete E6 open reading frame (ORF). Class 2 and class 3 transcripts are spliced within the E6 ORF and cannot encode a full-length E6 protein, but possibly code for a putative E6* protein. In order to selectively block E6 expression by RNAi, a 19 nucleotides (nt) long sequence within the HPV18 E6 intron, which is present only in class 1 transcripts, was chosen as siRNA target (Figure 1A, SEQ ID NO:4). This 19 nt target sequence, separated by a 9 nt spacer from its reverse complement sequence, was introduced into the siRNA generating pSUPER vector system, yielding pSUPER-18E6. The predicted secondary structure of the pSUPER-18E6 transcript is indicated in Figure 1B.

HPV18-positive HeLa cells were chosen for RNA and protein studies, since they reproducibly exhibited high DNA transfection efficiencies of more than 85% following calcium phosphate co-precipitation, thus allowing expression studies in transient transfections. As shown in Figure 1C, transfection of pSUPER-18E6 strongly reduced the levels of HPV18 E6/E7 transcripts in HeLa cells when compared to control cells transfected with basic expression vector pSUPER. In addition, the effect was specific, since E6/E7 transcript levels were not affected by siRNAs expressed from pSUPER constructs directed against the *P. pyralis* luciferase gene (Figure 1C, SEQ ID NO:6) or the p53 gene (not shown).

### Example 2: Targeting of E6 by siRNA specifically increases p53 and p21 levels

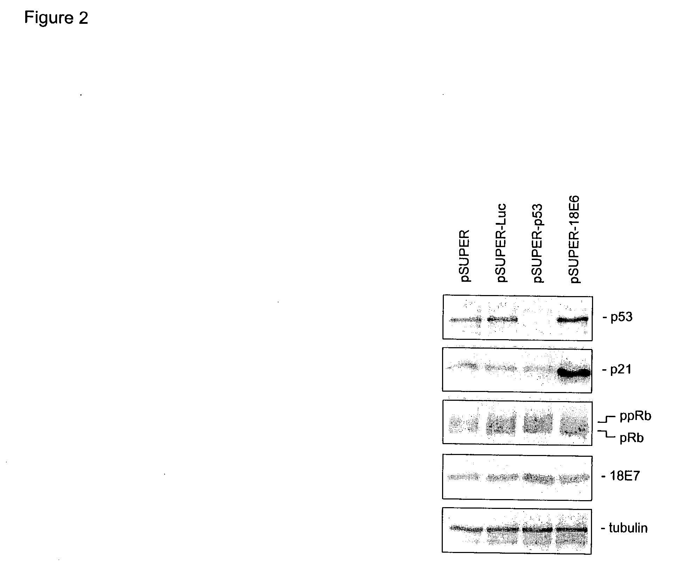

The effect of pSUPER-18E6 on E6 gene activities was assessed functionally, since there are no antibodies available that reliably detect the presumably very low amounts of endogenous E6 protein in HPV-positive cancer cells. The full-length E6 proteins, but not the putative truncated E6* forms (see above), of high risk HPVs induce the proteolytic degradation of p53. In line with a specific targeting of E6, p53 protein levels increased in HeLa cells following transfection with pSUPER-18E6, when compared to cells transfected with pSUPER or with pSUPER-Luc (Figure 2). In contrast, transfection of pSUPER-p53 strongly reduced p53 protein levels in HeLa cells.

The increase in p53 protein levels in the presence of pSUPER-18E6 was accompanied by a strong increase of p21 gene expression (Fig. 2), which is a transcriptional target for p53. This indicates that p53 not only accumulates but is also functionally active upon knockdown of E6 expression. Theoretically, increase of p21 activities should result in reduced phosphorylation of the retinoblastoma tumor suppressor protein, pRb, due to an increased functional inhibition of cyclin-dependent kinases. Indeed, analysis of the pRb protein showed a reduction of hyperphosphorylated forms and an increase in hypophosphorylated pRb in the presence of pSUPER-18E6 (Figure 2). Moreover, in support of selective targeting of E6, we found that pSUPER-18E6 did not affect the steady state levels of the HPV18 E7 protein (Figure 2). We conclude that pSUPER-18E6 can specifically inhibit E6 activities in HPV-positive cancer cells, without affecting E7 expression, leading to the activation of cellular pathways that are blocked by the E6 oncoprotein in untreated cells.

### Example 3: siRNA-mediated inhibition of HPV18 E6 can be rescued by E6 from oncogenic HPV

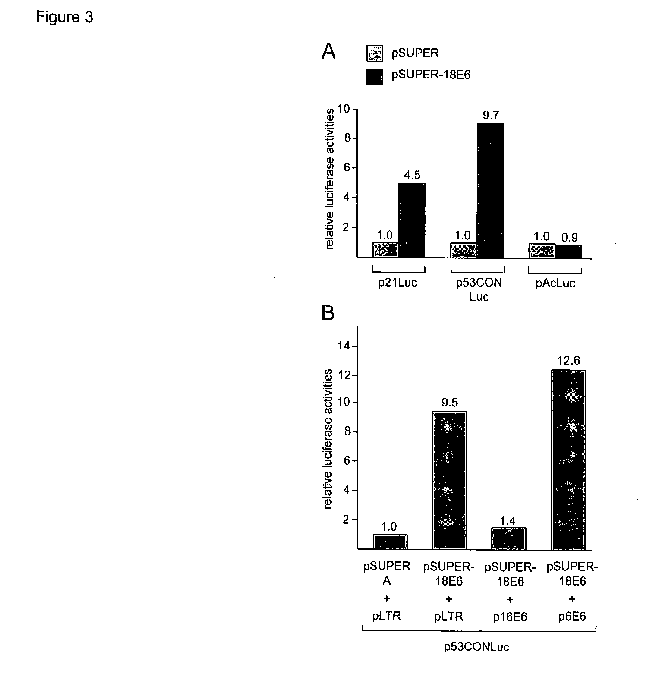

To further support the notion that pSUPER-18E6 restores dormant p53 activities by interfering with E6 expression, luciferase reporter assays were performed. Treatment of HeLa cells with pSUPER-18E6 resulted in the activation of reporter plasmids under the transcriptional control of the p21 promoter or of a synthetic p53 consensus recognition site upstream of a minimal promoter, but not of the human β-actin promoter, serving as a negative control (Figure 3A). Importantly, ectopic expression of the HPV16 E6 gene, which alike HPV18 E6 induces p53 degradation but does not bear the siRNA target sequence, counteracted the effects of pSUPER-18E6 in HeLa cells (Figure 3B). In contrast, ectopic expression of the HPV6 E6 gene, which does not induce p53 degradation, did not reverse the effects of pSUPER-18E6 (Figure 3B). Thus, RNA-mediated inhibition of HPV18 E6 can be specifically rescued by ectopic expression of a functional homologous E6 protein derived from another oncogenic HPV type, further demonstrating that the observed cellular effects are due to specific targeting of E6.

### Example 4: siRNA mediated targeting of E6 induces apoptosis in HPV-positive cells

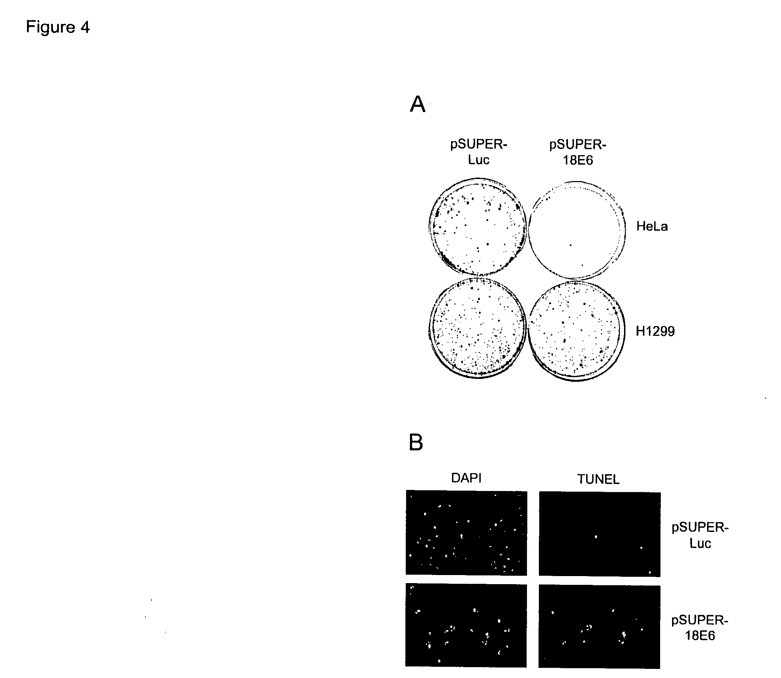

To study the cellular consequences of siRNA-mediated targeting of E6, we performed colony formation assays. To this end, cells were transfected with either pSUPER-18E6 or control vector pSUPER-Luc, together with pSV2Neo to allow selection for transfected cells by neomycin resistance. As shown in Figure 4A, pSUPER-18E6 had a dramatic effect on HPV18-positive HeLa cells, virtually completely abolishing their colony formation capacity. This effect was specific for HPV-positive cells, since the growth of HPV-negative cells, such as H1299 cells (Fig. 4A) or MeWo melanoma cells (not shown), was not affected by pSUPER-18E6.

In order to obtain insight into the molecular mechanisms that block the growth of pSUPER-18E6 transfected cells in colony formation assays, cells were investigated for signs of apoptosis by TdT-mediated dUTP biotin nick end labeling (TUNEL). As shown in Figure 4B, pSUPER-18E6-transfected HeLa cells exhibited a massive induction of apoptosis when compared to control-transfected cells, as visualized by a strong reduction in cell numbers, increased chromatin condensation, and a positive TUNEL assay. We conclude that siRNA directed against HPV18 E6 specifically kills HPV18-positive cancer cells with high efficiency, through the induction of apoptotic cell death.

### Example 5: Synthetic siRNAs that target the HPV16 and HPV18 E6 oncogenes efficiently induce apoptosis

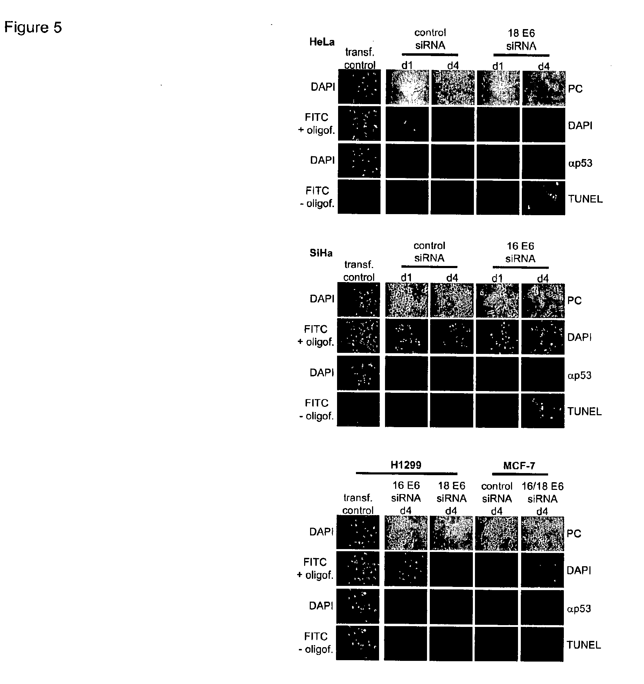

In contrast to our findings, a recent study has reported that synthetic siRNA directed against intron sequences of the HPV16 E6 gene induced only a marginal G1-arrest and no signs of apoptosis in HPV-positive tumor cell lines (Jiang and Milner, 2002). In order to investigate whether these different results may be due to the different virus types tested or to the different origins of the employed siRNAs (vector-borne versus chemically synthesized), we also analyzed the effects of synthetic siRNAs targeting the intron sequences within the HPV16 and HPV18 E6 genes, respectively (Figure 5, SEQ ID NOs: 2 and 7). In agreement with the results obtained with pSUPER-18E6, transfection of synthetic siRNAs targeting HPV16 E6 or HPV18 E6 resulted in an increase of p53 protein levels and a strong induction of apoptosis in HPV16- or HPV18-positive tumor cells, respectively (Figure 5). In contrast, HPV-negative control cells, such as MCF-7 mamma. carcinoma (wild-type p53) or H1299 lung cancer cells (p53 null) were not affected (Figure 5). Thus, both synthetic and vector-borne siRNAs that target the HPV16 and HPV18 E6 oncogenes are efficient inducers of apoptosis, selectively in HPV-positive tumor cells.

### MATERIALS AND METHODS

### Plasmids and synthetic siRNAs

Vector pSUPER generates biologically active siRNAs from the Pol III H1-RNA gene promoter (Brummelkamp *et al.,* 2002b). A synthetic double-stranded oligonucleotide (5'-CTAACACTGGGTTATACAAttcaagagaTTGTATAACCCAGTGTTAG-3', SEQ ID NO:4) was introduced into pSUPER, yielding pSUPER-18E6. Oligonucleotide sequences correspond to a 19 nt sequence from HPV18 E6 (nucleotides 385-403, numbering according to Accession number X05015 [Cole and Danos, 1987]) which are separated by a 9 nucleotide linker (lower case letters) from the reverse complement of the same 19 nt sequence. To generate control vector pSUPER-Luc, the double-stranded synthetic oligonucleotide 5'-CATCACGTACGCGGAATACttcaagagaGTATTCCGCGTACGTGATG-3' (SEQ ID NO:6) containing 19 nt derived from the *Photinus pyralis* (variant GL2) luciferase gene, separated by a 9 nucleotide linker from its reverse complement) was introduced into pSUPER. Vector pSUPER-p53 targets p53 transcripts and has been described before (Brummelkamp *et al.,* 2002b).
Synthetic siRNAs against mRNAs encoding HPV-16 E6 and HPV-18 E6, respectively, were obtained from Dharmacon Research (Lafayette, USA). The respective target sequence was for HPV-16 E6 5' UACAACAAACCGUUGUGUG (nucleotides 377-395 of Accession number K02718, SEQ ID NO:7), and for HPV-18 E6 5' CUAACUAACACUGGGUUAU (nucleotides 381-399 of Accession number X05015, SEQ ID NO:2).
For reporter gene analyses, the *P. pyralis* luciferase gene was under transcriptional control of a p53-consensus recognition site upstream of a minimal promoter in p53CONLuc, of the p21 promoter in p21Luc, and of the human β-actin promoter in pAcLuc. Plasmids p6E6 and p16E6 express the complete HPV16 and HPV6 E6 genes, respectively, from the Harvey murine sarcoma virus long terminal repeat; pLTR is the corresponding expression vector devoid of E6 inserts (Sedman *et al.,* 1991).

### Cells, Transfections and Luciferase Assays

HPV18-positive Hela cervical carcinoma cells, HPV16-positive SiHa cervical carcinoma cells, and HPV-negative MCF-7 mamma carcinoma, MeWo melanoma and H1299 lung cancer cells were all maintained in Dulbecco's minimal essential medium (pH 7.2), supplemented with 10% fetal calf serum. For protein and RNA analysis, exponentially growing cells were transfected in 10 cm petri dishes with 20 µg of the respective pSUPER-derived vector constructs by calcium-phosphate coprecipitation. Synthetic siRNAs (60 pmol per well of a 24 well plate) were transfected by Oligofectamine according to the manufacturer's instructions (Invitrogen, Karlsruhe, Germany).
For luciferase assays, cells were plated on 6 cm petri dishes and transfected with 2 µg of the respective luciferase reporter plasmids, 4 µg of either pSUPER or pSUPER-18E6, and 0.5 µg of the internal standard CMV-Gal, expressing the *E. coli* β-galactosidase gene under control of the human cytomegalovirus promoter, to correct for possible variations in transfection efficiencies. In order to analyze the effects of ectopically expressed E6, 1.5 µg p53CONLuc, 3 µg of either pSUPER or pSUPER-18E6, 0.5 µg of CMV-Gal, and 1.5 µg of either p6E6, p16E6, or pLTR were co-transfected, as detailed in the text. Cells were harvested 48 h after transfection, processed as described, and luciferase activites were quantitated using a Luci 1 microplate luminometer (Anthos, Krefeld, Germany).

### Protein and RNA analyses

Protein extracts were prepared 48 h after transfection, essentially as previously described (Butz *et al.,* 1995). For Western blot analyses, approximately 30 µg of protein was separated by 10% SDS-PAGE (except for pRb analyses where 100 µg of protein were separated by 7.5% SDS-PAGE) transferred to a Immobilon-P membrane (Millipore, Eschborn, Germany) and analyzed by enhanced chemiluminiscence (Amersham Biosciences, Freiburg, Germany) using anti-p21 antibody OP-64 (Calbiochem, Schwalbach, Germany), anti-p53 antibody DO-1 (Pharmingen, San Diego, USA), anti-pRb antibody G3-245 (Pharmingen), anti-HPV18 E7 antibody 18E7C (Butz *et al.,* 1998), and anti-tubulin antibody Ab-1 (Oncogene, Boston, USA).
For Northern blot analyses, RNA was isolated by guanidinium-thiocyanate-phenol-chloroform extraction. E6/E7 transcripts were detected using a radio-labeled 683 bp fragment covering the HPV18 E6/E7 region *(EcoR*I*-HindIII*-fragment), as described. Glycerylaldehyde-3-phosphate dehydrogenase (GAPDH) served as a control probe to monitor equal loading between individual lanes.

### Colony Formation Assays, Detection of Apoptosis

Cells were transfected with either pSUPER-18E6 or pSUPER-Luc, together with pSV2Neo to allow for the selection of transfected cells by neomycin resistance, in the presence of 1 mg/ml Geneticin (Invitrogen). Colonies were fixed with formalin and stained with crystal violet. For apoptosis detection, cells were split after transfection and grown on coverslips in the presence of 1 mg/ml Geneticin for 3 days. TdT-mediated dUTP biotin nick end labeling (TUNEL) analyses were performed, using the *in situ* cell death detection kit (Roche Molecular Biochemicals, Mannheim, Germany). In experiments with synthetic siRNAs, TUNEL analysis was performed 24-96 h after transfection. In addition, p53 was detected by the rabbit polyclonal antibody FL393 (Santa Cruz, Heidelberg, Germany) by immunofluorescence. Total DNA was stained with 4',6-diamidino-2-phenylindole (DAPI, Roche Molecular Biochemicals). Apoptotic strand breaks and total DNA were visualized by transmission epifluorescence microscopy.

### DESCRIPTION OF THE DRAWING

**Figure 1.** siRNA-targeting of HPV18 transcription. (A) Schematic illustration of the three classes of HPV transcripts expressed from the chromosomally integrated viral genomes in cervical cancer cells, and their coding potential. Coding regions are indicated by boxes, excised intron sequences are indicated by thin lines. Type 1 and type 2 transcripts are spliced to different cellular sequences which vary between individual HPV18-positive cancer cell lines. The sequence targeted by pSUPER-18E6 is schematically indicated by a thick line (marked by an asterisk) and is present only in class 1 transcripts which encode E6. HPV16-positive cancer cells exhibit a corresponding general transcription pattern, with the exception that alternative splicing within E6 can give rise to two putative E6 splice variants, E6* I and E6* II. (**B**) Predicted secondary structure of pSUPER-18E6 transcripts. (C) Down-regulation of HPV18 E6/E7 transcripts by pSUPER-18E6. GAPDH: detection of glycerylaldehyde-3-phosphate dehydrogenase transcripts. pSUPER: basic expression vector without an siRNA-encoding insert; pSUPER-Luc: pSUPER expressing siRNA targeting the *P. pyralis* luciferase gene.

**Figure 2.** Targeting of E6 leads to the restoration of p53-associated pathways in HPV-positive cancer cells. pSUPER-18E6 increases p53 protein levels in HeLa cells. This is linked to augmented p21 protein levels and an increase in hypophosphorylated pRb (pRb, hypophosphorylated; ppRb, hyperphosphorylated forms). HPV18 E7 levels are unaffected by pSUPER-18E6. Tubulin: detection of tubulin protein to monitor equal loading between individual lanes. pSUPER-p53: control vector producing siRNA targeting p53 transcripts (Brummelkamp *et al.,* 2002b).

**Figure 3.** Reactivation of p53-mediated transcriptional stimulation in HeLa cells by siRNA-targeting of E6. (A) pSUPER-18E6 stimulates transcription of the p21 promoter (p21 Luc) and a minimal promoter under control of a p53 consensus recognition site (p53CONLuc), but not of the human β-actin promoter (pAcLuc), serving as a negative control. Indicated are luciferase values relative to the activities of the individual promoters in the presence of basic vector pSUPER (arbitrarily set at 1.0). Values represent the means of at least two independent experiments, each performed in triplicate. Results from individual transfections varied by less than 15%. (**B**) Ectopic expression of HPV16 E6 reverses the effects of pSUPER-18E6. p53CONLuc activities in the presence of basic vectors pSUPER and pLTR were set at 1.0. The increase of p53CONLuc activities in the presence of pSUPER-18E6 was counteracted by ectopically expressed HPV16 E6, but not by HPV6 E6.

**Figure 4**. pSUPER-18E6 selectively blocks the growth of HPV18-positive cancer cells by inducing apoptosis. (**A**) Colony formation assays of HeLa cells (HPV18-positive) and H1299 cells (HPV-negative), transfected with the E6-targeting vector pSUPER-18E6 or control vector pSUPER-Luc, respectively. (**B**) E6-targeting by pSUPER-18E6 induces massive apoptosis in HPV18-positive cells. HeLa cells were transfected with control vector pSUPER-Luc (upper panels) or pSUPER-18E6 (lower panels), together with pSV2Neo, and grown for 3 days in the presence of Geneticin to select for transfected cells. Total DNA was stained with DAPI, cells undergoing apoptosis were visualized by TUNEL analysis. To avoid confluency of the control cells, a five-fold lower number of pSUPER-transfected cells was plated for this figure.

**Figure 5**. Synthetic siRNAs against E6 induce p53 accumulation and apoptosis in HPV-positive cancer cell lines. siRNAs specific for HPV-18 E6 (HeLa, H1299, MCF-7) or HPV-16 E6 (SiHa, H1299, MCF-7) were transfected into the cell lines indicated. As control, cells were transfected with the sense strand of the respective siRNA. As a measure of transfection efficiency, cells were transfected with FITC-dextran as indicated. Levels of p53 and induction of apoptosis, respectively, were determined 24 h (d1) and 96 h (d4) after transfection by fluorescence analysis. In addition, cells were visualized by phase contrast microscopy (PC) and nuclei by DAPI.

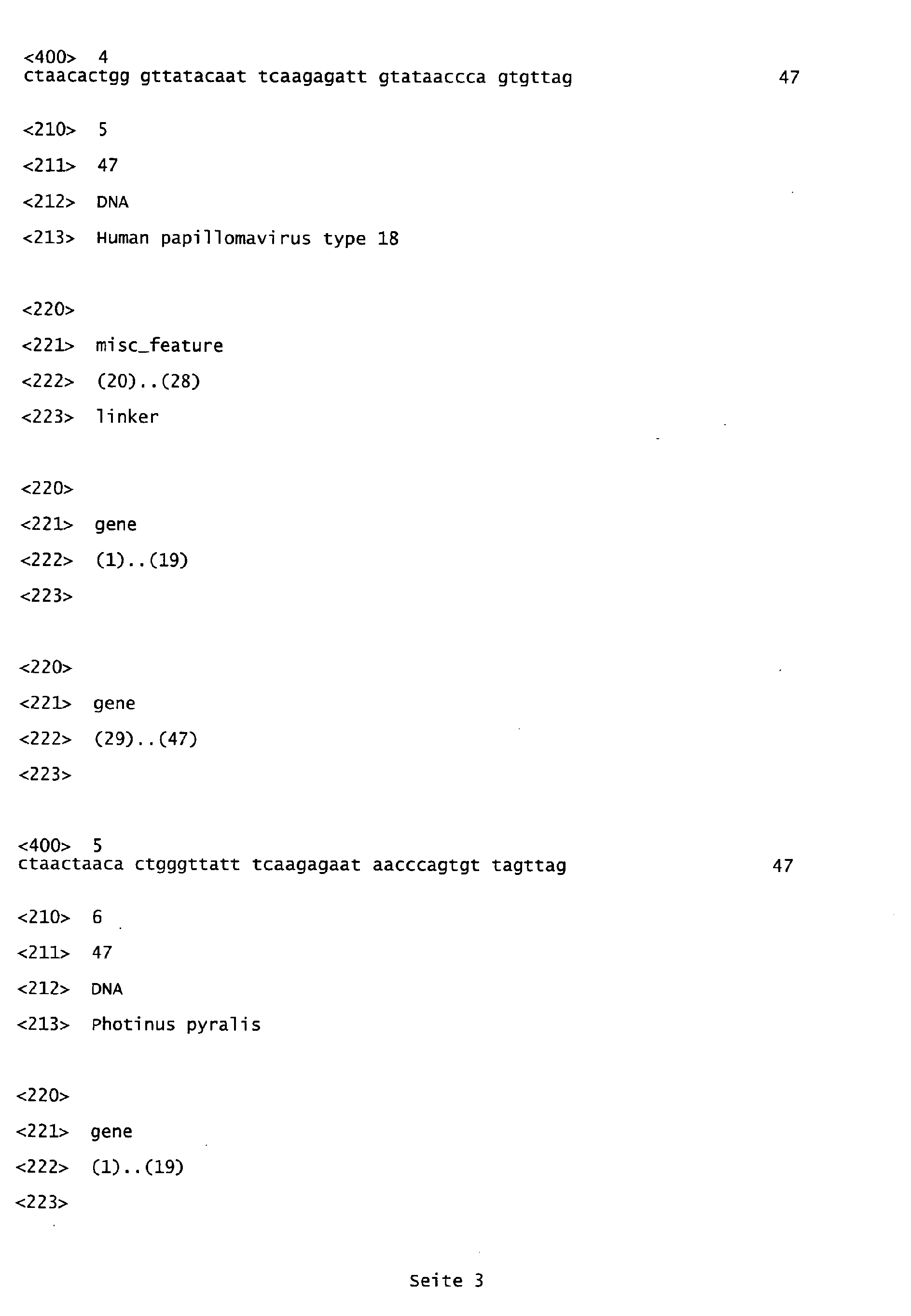

## Claims

1. The use of a nucleic acid containing the sequence of SEQ ID NO:1, a fragment or derivative thereof, to prepare siRNA which induces apoptosis in HPV18-infected cells.

2. The use according to claim 1, wherein said nucleic acid has a length of 15 to 25 nucleotides.

3. The use according to claim 1 or 2, wherein said nucleic acid has a length of 19 to 22 nucleotides.

4. The use according to any of claims 1 to 3, wherein said nucleic acid has a length of 19 nucleotides.

5. The use according to any of claims 1 to 4, wherein said nucleic acid comprises the sequence of SEQ ID NO:2, a fragment or derivative thereof.

6. The use according to any of claims 1 to 4, wherein said nucleic acid comprises the sequence of SEQ ID NO:3, a fragment or derivative thereof.

7. The use of a nucleic acid containing the sequence of SEQ ID NO:7, a fragment or derivative thereof, to prepare siRNA which induces apoptosis in HPV16-infected cells.

8. The use according to any of claims 1 to 7, wherein the siRNA is delivered into a HPV16- and/or HPV18-infected target cell.

9. The use according claim 8, wherein the delivery is done by using liposomes.

10. The use of a nucleic acid containing the sequence of SEQ ID NO:4, a fragment or derivative thereof, to prepare siRNA which induces apoptosis in HPV18-infected cells.

11. The use of a nucleic acid containing the sequence of SEQ ID NO:5, a fragment or derivative thereof, to prepare siRNA which induces apoptosis in HPV18-infected cells.

12. The use of a nucleic acid containing the sequence of SEQ ID NO:8, a fragment or derivative thereof, to prepare siRNA which induces apoptosis in HPV16-infected cells.

13. The use according to any of claims 10 to 12, wherein the nucleic acid is inserted into an expression vector.

14. The use according to claim 13, wherein the expression vector allows for the production of dsRNA.

15. The use according to claim 13 or 14, wherein the expression vector is pSUPER.

16. An expression vector containing the sequence of SEQ ID NO:4, a fragment or derivative thereof, SEQ ID NO:5, a fragment or derivative thereof, or SEQ ID NO:8, a fragment or derivative thereof.

17. The use according to any of claims 13 to 15, wherein the expression vector is delivered into a HPV 18- or a HPV16-infected target cell.

18. The use according to claim 17, wherein the delivery is done by using translocating peptides.

19. A nucleic acid containing the sequence of SEQ ID NO:1, 2, 3, 4, 5, 7 or 8 for use as a pharmaceutical.

20. The use of a nucleic acid containing the sequence of SEQ ID NO: 1, 2, 3, 4, 5, 7, or 8, optionally in combination with an active apoptosis-inducing compound, for the manufacture of a medicament for the treatment of HPV16- and/or HPV-18-related diseases selected from a group consisting of Condylomata acuminata, Condylomata plana, Condylomata gigantea, Cervical Intraepithelial Neoplasia, Bowenoid papulosis, larynx papilloma, spinalioma, Morbus Bowen of the skin, penis carcinoma, vulva carcinoma, cervical carcinoma, larynx carcinoma, oropharynx and tongue carcinoma.

21. The use according to claim 20, wherein the of HPV16- or HPV-18-related tumor is cervical carcinoma.

22. A medicament for the treatment of HPV16- and/or HPV-18-related diseases, comprising a nucleic acid containing the sequence of SEQ ID NO:1, 2, 3, 4, 5, 7, or 8, optionally an active compound, and a pharmaceutically acceptable carrier.


SEQUENCE LISTING

<110> Deutsches Krebsforschungszentrum

<120> HPV-E6-specific siRNAs for the treatment of HPV-related cancers

<130> DK62109EP

<160> 8

<170> PatentIn version 3.1

<210> 1
<211> 15
<212> RNA
<213> Human papillomavirus type 18

<220>
<221> gene
<222> (1)..(15)
<223>

<400> 1
cuaacacugg guuau 15

<210> 2
<211> 19
<212> RNA
<213> Human papillomavirus type 18

<220>
<221> gene
<222> (1)..(19)
<223>

Seite 1

<400>  2
cuaacuaaca cuggguuau                                                    19

<210>  3

<211>  19

<212>  RNA

<213>  Human papillomavirus type 18


<220>

<221>  gene

<222>  (1)..(19)

<223>


<400>  3
cuaacacugg guuauacaa                                                    19

<210>  4

<211>  47

<212>  DNA

<213>  Human papillomavirus type 18


<220>

<221>  gene

<222>  (1)..(19)

<223>


<220>

<221>  misc_feature

<222>  (20)..(28)

<223>  linker


<220>

<221>  gene

<222>  (29)..(47)

<223>


Seite 2

<220>

<221>  misc_feature

<222>  (20)..(28)

<223>  linker


<220>

<221>  gene

<222>  (29)..(47)

<223>


<400>  6
catcacgtac gcggaatact tcaagagagt attccgcgta cgtgatg                    47

<210>  7

<211>  19

<212>  RNA

<213>  Human papillomavirus type 16


<220>

<221>  gene

<222>  (1)..(19)

<223>


<400>  7
uacaacaaac cguugugug                                                   19

<210>  8

<211>  47

<212>  DNA

<213>  Human papillomavirus type 16


<220>

<221>  gene

<222>  (1)..(19)

<223>


Seite 4

<220>

<221>  misc_feature

<222>  (20)..(28)

<223>  linker


<220>

<221>  gene

<222>  (29)..(47)

<223>


<400>  8
tacaacaaac cgttgtgtgt tcaagagaca cacaacggtt tgttgta                    47